# EUROPEAN PATENT SPECIFICATION

(11) **EP 0 624 642 B1**
(45) Date of publication and mention of the grant of the patent: **20.01.1999**
(21) Application number: 93107758.0
(22) Date of filing: 12.05.1993
(51) Int. Cl.: C12N 9/54, A61K 38/48

(54) **Novel thrombolytic agent, process for preparing same and its use for the preparation of a thrombi dissolving medicament**
Neues thrombolytisches Agens, Verfahren zu seiner Herstellung und seines Gebrauches für die Herstellung eines thrombenauflösenden Arzneimittels
Agent thrombolitique nouveau, procédé pour sa préparation et son utilisation pour la préparation d'un médicament dissolvant des caillots

(43) Date of publication of application: 17.11.1994
(73) Proprietor: INDIAN COUNCIL FOR MEDICAL RESEARCH, New Delhi 110 029 (IN)
(72) Inventor: Balaraman, Kothandapani, Pondicherry-605-606 Indira Nagar (IN); Kuppusamy, Musavan, Pondicherry-605-606 Indira Nagar (IN)
(74) Representative: Andrae, Steffen, Dr.

(56) References cited:
- EP-A- 0 430 637
- AGRICULTURAL AND BIOLOGICAL CHEMISTRY. vol. 41, no. 5 , 1977 , TOKYO JP pages 745 - 754 K. YOSHIDA ET AL 'Purification and some properties of Bacillus sphaericus protease'
- CHEMICAL ABSTRACTS, vol. 114, no. 21, 27 May 1991, Columbus, Ohio, US; abstract no. 203257, S. VYBORNYKH ET AL 'Screening of bacillar proteases with the plasmin and activator types of their action of fibrin' page 439 ; & MIKROBIOLOGIYA vol. 60, no. 1 , 1991 pages 93 - 100
- CHEMICAL ABSTRACTS, vol. 106, no. 9, 2 March 1987, Columbus, Ohio, US; abstract no. 64089, A. BONDARCHUK ET AL 'Physicochemical properties of proteolytic enzymes of different strains of the genus Bacillus' page 323 ; & MIKROBIOL. ZH. vol. 48, no. 5 , 1986 , KIEV pages 7 - 10
- PATENT ABSTRACTS OF JAPAN vol. 10, no. 366 (C-390)(2423) 6 December 1986 & JP-A-61 162 184 (NORIYOSHI MORIMOTO) 22 July 1986

## Description

This invention relates to a process for the preparation of thrombinase. Thrombinase is a thrombi dissolving agent containing a fibrinolytic enzyme having an advantageous application for the treatment of cerebral thrombosis, myocardial infarction, deep vein thrombosis and in the prevention of post surgical adhesion.

Streptokinase (from Streptococci), Urokinase (from human urine) and Tissue Plasminogen Activator, (TPA, from human tissue culture) are the main thrombolytic agents currently known in the art. Streptokinase-aspirin combinations provide a significant relief even if the treatment is commenced 24 hrs after a heart attack. Prourokinase, also known as kidney plasminogen activator, is designed to dissolve clots in the blood without interfering with the body's normal blood clotting process. Beecham Laboratories clot dissolving product, an isolated plasminogenstreptokinase activator complex, trade named, Eminase, is claimed to have reduced the death rate in heart attack victims by 50%. Genentech's TPA (Activase) is also highly effective in dissolving blood clots. Urokinase, Streptokinase and TPA, the first plasminogen activators available for clinical use, activate the plasminogen to active plasmin. The actual clot lysis is carried out by the active plasmin. The action of plasmin, however, is not restricted to the lysis of fibrin present in thrombus; plasmin also attacks fibrinogen and other clotting factors, which can lead to severe bleeding. Fibrinolytic potency and specificity of these agents are markedly enhanced by covalently linking them to a fibrin specific antibody. This type of targetting the fibrinolytic agent to the site of thrombi opens up the possibility of using direct acting fibrinolytic enzymes in the place of plasminogen activators.

It is an object of this invention to propose a novel process for the preparation of thrombinase.

It is another object of this invention to propose such an improved process which will utilize material now discarded as waste in the known fermentation processes of Bacillus Sphaericus Serotype H5a and H5b.

It is a still further object of this invention to propose such a process which will employ the waste or discarded material in employ the above conventional process to produce useful chemicals as a by-product in an economical manner so that the known main fermentation itself becomes more economical and productive.

It is thus a still further object of this invention to propose an improved process for the production of biopesticide.

In known fermentations of Bacillus Sphaericus Serotype H5a and H5b there is obtained a cell free culture filtrate. This is generally discarded and is thus treated as waste.

We turned our attention to investigating the filtrate now being discarded to find out whether any valuable chemical is present therein and whether such a chemical, if any, can be economically recovered to give a by-product so as to make the main production of biopesticide economical.

We tried the effect of this filtrate on several protein sources e. g. casein and bovine serum, albumin mainly for protease activity. It was observed that it could liquefy blood clots. Further investigation indicated that it did not affect the red blood cells and acted only upon the fibrin.

This confirmed our above observations and that it is an enzyme, particularly a protease.

Attention was therefore directed into preparing a useful drug from this cell free filtrate.

It is known from Agricultural and Biological Chemistry, Vol. 41, No. 5, 1977, Tokyo, pp.745-754 that a culture filtrate of a strain of Bacillus sphaericus can contain a protease. The protease described in said reference has maximal caseinolytic activities around pH 9,0 to 9,3, and its molecular weight was estimated by gelchromatography as being 27,000.

That some other types of bacteria of the genus Bacillus produce enzymes with plasmin type activities is known from two Russian publications according to Chemical Abstracts, vol. 114, no. 21, abstract no. 203257 and vol. 106, no.9, abstract no. 64089 respectively.

From the nature of the protease identified by us, based on the known activities and published literature, we could conclude that a useful thrombolytic agent could be manufactured from this enzyme (protease)

Several investigations were carried out using different sources of the cell free filtrate and we could develop a simple physico-chemical process to manufacture the thrombolytic agent.

In general, our process comprises subjecting the culture filtrate obtained from the main process of conventional submerged fermentation of Bacillus Sphaericus Serotype H5a and H5b to simple physico-chemical steps comprising ultrafiltration of the cell free filtrate, concentration of the same and salting out all protein from the said cell free filtrate using ammonium sulphate substantially and decolourizing the protein in liquid medium.

The decolourized protein obtained can be further subjected to dialysis and lyophilization to obtain a crude powder which may be subjected to further purification.

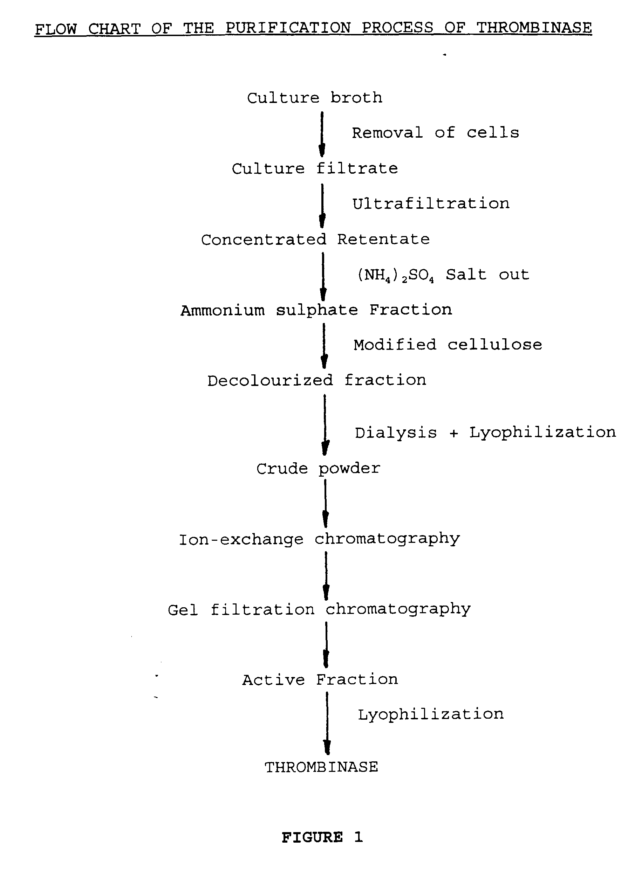

The general process steps are illustrated in the following flow chart enclosed as Figure 1. The invention will now be more fully described with reference to the following examples:

It should be noted that the salting out by using ammonium sulphate is only to change the solubility of the macromolecules of the filtrate.

We give below a table showing the details of the filtrate before it was subjected to salting out of protein and after it was subjected to salting out of protein.

| S1. No. | Parameter | Before salting out | After salting out |
|---|---|---|---|
| 1. | Physical appearance of filtrate | liquid | liquid |
| 2. | Density | 1.1 | 1.05 |
| 3. | % of solid | 0.05 | 0.05 |
| 4. | pH | 7 | 5.5 |
| 5. | colour | Dark brown | Dark brown |

The protein that has been salted out from the filtrate is converted into an aqueous solution and then subjected to decolourization using commercially available modified cellulose such as cell debris removers supplied by Whatman or DE 52 ion exchange resin from the same manufacturer.

### SUITABLE OTHER FORMS OF DE-COLOURIZATION AGENTS MAY BE STUDIED.

### Dialysis and Lyophilization

These two steps are carried out in the following manner: The precipitate obtained after salting out is dissolved in water, loaded into tubular dialysing membrane bags of molecular weight cut off limit 10,000 and dialysed overnight against distilled water.

### Purification of the crude powder obtained after lyophilization

The crude powder obtained is subjected to a two-stage purification.

In the first stage the powder is made into a solution with water and subjected to ion exchange treatment in the following manner: The decolourized fraction was loaded on Q-sepharose column and eluted in two steps using Tris-HCl buffer, pH 8.0 containing 0.1 M and 0.5 M NaCl concentration respectively. The unbound fraction eluted with 0.1M NaCl concentration contained the fibrinolytic protease activity.

The second purification is carried out by gel filtration in the following manner: The active fraction obtained after Q-sepharose chromatography was further purified on Sephacryl S300 column. Elution was carried out with 0.01 M Tris-HCl buffer, pH 8. Two fractions were obtained among which the second fraction contained the fibrinolytic enzyme.

### Purification of the Crude Powder

Types of ion exchange used for first stage purification are to be specified and the extent of purification may be stated.

Second stage purification may be made more definitive by clarifying the gel filtration, types of gel, specifications of gel, stages and final purity achieved.

### Product Description

The product is a novel thrombi dissolving agent containing a fibrinolytic enzyme (Thrombinase) isolated, identified and purified for the first time from Bacillus Sphaericus. This product has potential use for the treatment of cerebral thrombosis stroke, moycardial infarction, deep vein thrombosis and in the prevention of post surgical adhesions. The preparation can be used on its own or in combination with specific antibodies. Though the product is not a plasminogen activator, it acts very specificallly on fibrin clots. The fibrinolytic action is greater than that of streptokinase and urokinase.

It has optimum activity in the range of 7-9 pH and is stable over the pH range 5-7.5. Molecular weight (gel filtration) was estimated to be around 18,500. It is stable below 40°C and retains 80% or more activity on standing for 24 hr at 30°C in pH 7.2 Tris-HCl buffer.

### Biological activity

### Assay for fibrinolytic potency

Fibrinolytic activity of the purified enzyme was evaluated in comparison to standard streptokinase according to the modified method of Astrup und Mullertz (1952).

Fibrinolytic potency was estimated by measuring the extent of liquefication (zone diameter) produced by the enzyme on fibrin clot of fibrinogen, plasminogen and thrombin at 7.5 pH and 37°C in 15 min. Streptokinase, the reference standard, was diluted to contain 500 units/ml. Thrombinase was dissolved in Tris-HCl buffer pH 7.5 to contain 1 mg protein/ml. Quantities of 20 and 40 µl of streptokinase and the test preparation were placed as a droplet on the surface of the fibrin clot and incubated at 37°C. The diameter of the digested area was measured after 30 minutes of incubation .

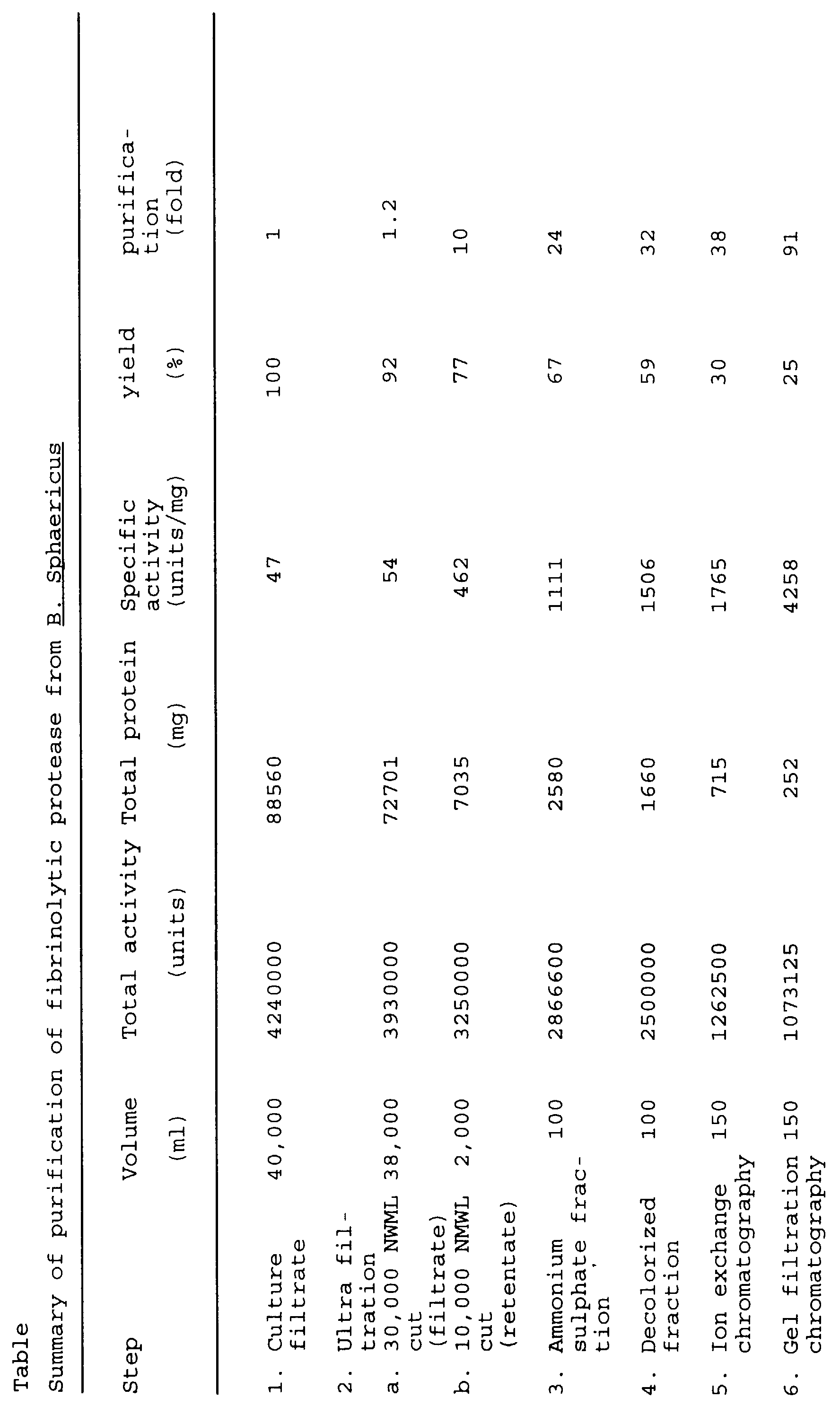

## Claims

1. A thrombolytic agent which can be obtained from a cell-free culture filtrate of fermentations of Bacillus Sphaericus serotype H5a and H5b and which has an optimum enzymatic activity range from pH 7 to 9 and a molecular weight (gel filtration) of about 18.500.

2. A process for the preparation of a novel thrombolytic agent according to claim 1, which comprises
- obtaining a cell-free culture filtrate from the known culture broth of the fermentation of Bacillus Sphaericus Serotype H5a and H5b,
- subjecting said cell-free culture filtrate to a step of ultrafiltration in order to obtain a concentrated retentate,
- followed by salting out substantially all protein from said concentrate using a salting agent such as ammonium sulphate,
- converting the salted out protein into an aqueous solution and subjecting said solution to decolourization using a modified cellulose,
- subjecting the decolourized fraction to dialysis and lyophilization to obtain a crude powder of said protein,
- followed by preparing a solution of said crude powder and subjecting same to a chromatographic treatment to obtain a purified material, and
- finally lyophilizing said purified material to obtain the thrombolytic agent.

3. A process as claimed in claim 2, wherein the modified cellulose is a commercially available product called "DE52" supplied by Whatman Biosystems Ltd., England.

4. A process as claimed in claim 2 or 3, wherein the dialysis and lyophilization of the cellulose treated fraction is carried out by using a semi-permeable membrane (10000 daltons cut off) and a lyophilizer respectively to obtain said crude powder of the protein.

5. A process as claimed in any of the claims 2 to 4, wherein the chromatographic treatment is carried out in two stages v.i.z. a first stage of ion exchange chromatography and a second stage of gel filtration chromatography.

6. A process as claimed in claim 5, wherein the first stage of ion exchange chromatography is carried out by subjecting a solution of the crude powder to Q Sepharose (Pharmacia Ltd., Sweden) anion exchange chromatography using 0.01 M Tris-HCl buffer, pH 8, containing 0.001 M CaCl₂ and stepwise elution using the same buffer containing 0.1 M NaCl and 0.5 M NaCl respectively.

7. A process as claimed in claim 5 or 6, wherein the second stage gel filtration chromatography is carried out by gel filtration on Sephacryl S3000 (Pharmacia Ltd., Sweden) using 0.05 M Tris-HCl buffer, pH 7.5, containing 0.1 M NaCl.

8. A process as claimed in any of the claims 2 to 7, wherein the final step is carried out by subjecting the dialysed positive fractions after an additional gel filtration chromatography to lyophilization.

9. Use of a thrombolytic agent according to claim 1 or of the product of the process of any of the claims 2 to 8 for the preparation of a thrombi dissolving medicament.

## Patentansprüche

1. Thrombolytikum, das aus einem zellfreien Kulturfiltrat von Fermentationen von Bacillus Sphaericus Serotyp H5a und H5b erhalten werden kann und das einen optimalen enzymatischen Aktivitätsbereich von pH 7 bis 9 sowie ein Molekulargewicht (Gelfiltration) von etwa 18500 aufweist.

2. Verfahren zur Herstellung eines neuen Thrombolytikums nach Anspruch 1, das umfaßt,
- Gewinnen eines zellfreien Kulturfiltrats der bekannten Kulturbrühe der Fermentation von Bacillus Sphaericus Serotyp H5a und H5b,
- Unterwerfen des genannten zellfreien Kulturfiltrats einer Ultrafiltrationsstufe, um ein konzentriertes Retentat zu gewinnen,
- gefolgt von einem im wesentlichen vollständigen Aussalzen des Proteins aus dem genannten Konzentrat unter Verwendung eines Aussalzmittels wie Ammoniumsulfat,
- Überführen des ausgesalzten Proteins in eine wässrige Lösung und Entfärben dieser Lösung unter Verwendung einer modifizierten Zellulose,
- Dialysieren und Lyophilisieren der entfärbten Fraktion, um ein rohes Pulver des genannten Proteins zu gewinnen, gefolgt von der Herstellung einer Lösung des genannten rohen Pulvers und einer chromatographischen Behandlung derselben, um ein gereinigtes Material zu gewinnen, und
- abschließendes Lyophilisieren des gereinigten Materials, um das Thrombolytikum zu gewinnen.

3. Verfahren nach Anspruch 2, bei dem die modifizierte Zellulose ein kommerziell erhältliches Produkt mit der Bezeichnung "DE52", vertrieben von Whatman Biosystems Ltd., England, ist.

4. Verfahren nach Anspruch 2 oder 3, bei dem die Dialyse und die Lyophilisierung der Zellulose-behandelten Fraktion dadurch ausgeführt wird, daß man eine semipermeable Membran (Ausschlußgrenze: 10000 Dalton) bzw. ein Lyophilisiergerät verwendet, um das genannte rohe Pulver des Proteins zu erhalten.

5. Verfahren nach irgendeinem der Ansprüche 2 bis 4, bei dem die chromatographische Behandlung in zwei Stufen durchgeführt wird, und zwar mit einer ersten Ionenaustauschchromatographie-Stufe und einer zweiten Gelfiltrationschromatographie-Stufe.

6. Verfahren nach Anspruch 5, bei dem die Ionenaustauschchromatographie der ersten Stufe so durchgeführt wird, daß man eine Lösung des rohen Pulvers einer Q-Sepharose (Pharmacia Ltd., Schweden)-Anionenaustauschchromatographie unter Verwendung eines 0,01 M Tris-HCl-Puffers, pH 8, mit einem Gehalt an 0,001 M CaCl₂ unterwirft und unter Verwendung des gleichen Puffers mit einem Gehalt an 0,1 M NaCl bzw. 0,5 M NaCl schrittweise eluiert.

7. Verfahren nach Anspruch 5 oder 6, bei dem die Gelfiltrationschromatographie in der zweiten Stufe als Gelfiltration auf Sephacryl S 3000 (Pharmacia Ltd., Schweden) unter Verwendung eines 0,5 M Tris-HCl-Puffers, pH 7,5, mit einem Gehalt an 0,1 M NaCl durchgeführt wird.

8. Verfahren nach irgendeinem der Ansprüche 2 bis 7, bei dem die abschließende Stufe so durchgeführt wird, daß man die dialysierten positiven Fraktionen nach einer zusätzlichen Gelfiltrationschromatographie lyophilisiert.

9. Verwendung eines Thrombolytikums nach Anspruch 1 oder des Produkts des Verfahrens nach irgendeinem der Ansprüche 2 bis 8 zur Herstellung eines Medikaments zur Auflösung von Thromben.

## Revendications

1. Agent thrombolytique qui peut être obtenu à partir d'un filtrat de culture acellulaire de fermentations des sérotypes H5a et H5b de Bacillus Sphaericus et qui possède un domaine d'activité enzymatique optimale dans la fourchette de pH de 7 à 9 et un poids moléculaire (filtration sur gel) d'environ 18.500.

2. Procédé pour la préparation d'un nouvel agent thrombolytique selon la revendication 1, qui comprend le fait de:
- se procurer un filtrat de culture acellulaire à partir du bouillon de culture connu de la fermentation des sérotypes H5a et H5b de Bacillus Sphaericus,
- soumettre ledit filtrat de culture acellulaire à une étape d'ultrafiltration dans le but d'obtenir un rétentat concentré,
- relarguer ensuite essentiellement toutes les protéines dudit concentrat en utilisant un agent de relargage tel que le sulfate d'ammonium,
- convertir les protéines relarguées en une solution aqueuse et soumettre ladite solution à une décoloration en utilisant une cellulose modifiée,
- soumettre la fraction décolorée à une dialyse et à une lyophilisation pour obtenir une poudre brute desdites protéines,
- préparer ensuite une solution de ladite poudre brute et la soumettre à un traitement chromatographique pour obtenir une matière purifiée, et
- enfin, lyophiliser ladite matière purifiée pour obtenir l'agent thrombolytique.

3. Procédé selon la revendication 2, dans lequel la cellulose modifiée est un produit disponible dans le commerce appelé "DE52" fourni par Whatman Biosystems Ltd., Angleterre.

4. Procédé selon la revendication 2 ou 3, dans lequel on effectue la dialyse et la lyophilisation de la fraction traitée avec la cellulose en utilisant une membrane semi-perméable (coupure de 10.000 daltons) et un agent de lyophilisation, respectivement, pour obtenir ladite poudre brute des protéines.

5. Procédé selon l'une quelconque des revendications 2 à 4, dans lequel on effectue le traitement chromatographique en deux étapes, c'est-à-dire une première étape de chromatographie par échange d'ions et une seconde étape de chromatographie par filtration sur gel.

6. Procédé selon la revendication 5, dans lequel on effectue la première étape de la chromatographie par échange d'ions en soumettant une solution de la poudre brute à une chromatographie par échange d'anions sur une colonne Q Sepharose (Pharmacia Ltd., Suède) en utilisant un tampon Tris-HCl 0,01 M, pH 8, contenant du CaCl₂ 0,001 M et a une élut ion par paliers en utilisant le même tampon contenant du NaCl 0,1 M et du NaCl 0,5 M, respectivement.

7. Procédé selon la revendication 5 ou 6, dans lequel on effectue la seconde étape de chromatographie par filtration sur gel via une filtration sur gel sur un colonne Sephacryl S3000 (Pharmacia Ltd., Suède) en utilisant un tampon Tris-HCl 0,05 M, pH 7,5, contenant du NaCl 0,1 M.

8. Procédé selon l'une quelconque des revendications 2 à 7, dans lequel on effectue l'étape finale en soumettant à une lyophilisation les fractions positives issues de la dialyse après une chromatographie supplémentaire par filtration sur gel.

9. Utilisation d'un agent thrombolytique selon la revendication 1 ou du produit du procédé selon l'une quelconque des revendications 2 à 8, pour la préparation d'un médicament dissolvant les thrombi.
